# EUROPEAN PATENT SPECIFICATION

(11) **EP 0 853 949 B1**
(45) Date of publication and mention of the grant of the patent: **09.04.2003**
(21) Application number: 97250375.9
(22) Date of filing: 18.12.1997
(51) Int. Cl.: A61L 17/00

(54) **Process for treating hydrolyzable resorbable surgical suture material**
Verfahren zum Behandeln von chirugischem Nahtmaterial
Procédé de traitment de matériaux de suture chirugicales hydrolysables

(30) Priority: 16.01.1997 DE 19702708
(43) Date of publication of application: 22.07.1998
(73) Proprietor: Ethicon GmbH, 22851 Norderstedt (DE)
(72) Inventor: Hinsch, Bernhard, Dr., 22851 Norderstedt (DE); Hündorf, Uwe, Dr., 24558 Henstedt-Ulzburg (DE); Ruthenberg, Jürgen, Dipl.-Ing., 22175 Hamburg (DE)
(74) Representative: UEXKÜLL & STOLBERG

(56) References cited:
- BROWNING A. ; CHU C. C.: "The effect of annealing treatments on the tensile properties and hydrolytic degradative properties of polyglycolic acid" JOURNAL OF BIOMEDICAL MATERIALS RESEARCH, vol. 20, no. 5, May 1986 - June 1986, pages 613-632, XP002097446 US
- ATHANASIOU K A ET AL: "Sterilization, toxicity, biocompatibility and clinical applications of polylactic acid/polyglycolic acid copolymers" BIOMATERIALS, vol. 17, no. 2, 1996, page 93-102 XP004032807
- DATABASE WPI Section Ch, Week 9712 Derwent Publications Ltd., London, GB; Class A23, AN 97-128813 XP002097126 & JP 09 012688 A (TOYOBO KK) , 14 January 1997
- CHU C. C.: "A comparison of the effect of pH on the biodegradation of two synthetic sutures" ANNALS OF SURGERY, vol. 195, no. 1, January 1982, pages 55-59, XP002097448 US
- CHU C. C. ; WILLIAMS S. F.: "The effect of gamma irradiation on the enzymatic degradation of polyglycolic acid absorbable sutures" JOURNAL OF BIOMEDICAL MATERIALS RESEARCH, vol. 17, no. 6, 13 November 1983, pages 1029-1040, XP002097447 US

## Description

The invention relates to a process for treating hydrolyzable resorbable surgical suture material.

After insertion into the body, resorbable surgical sutures are resorbed in course of time. Generally, the tensile strength has already significantly descreased before considerable resorption starts.

Polyglactin 910 has proved to be a good resorbable surgical suture material. Polyglactin 910 is a copolymer of glycolide and lactide in the ratio 9:1, which is sold by the applicant under the trademark "Vicryl". Polyglactin 910 is degraded by hydrolysis. The resulting degradation products, glycolic acid and lactic acid, are metabolized in the body. Surgical suture material made of polyglactin 910 can include monofilament or braided threads. Braided threads are preferably coated.

For indications in which a suture has served its purpose after a few days, a suture material having a resorption period which is shorter than that of conventional resorbable suture materials is advantageous.

In order to shorten the resorption period of resorbable suture material it is known to irradiate the suture material during the manufacture, e.g., by means of ⁶⁰Co gamma irradiation. This produces defects in the polymer structure of the suture material which result in an accelerated decrease of the tensile strength and a shortened resorption period in vivo after implantation of the suture material. The irradiation during manufacture, however, for reasons of equipment, can be performed without high expenditure under pre-determined, strict conditions only, which makes it impossible to adjust the resorption properties of the suture material in a flexible manner.

In A. Browning and C.C. Chu: "The effect of annealing treatments on the tensile properties and hydrolytic degradative properties of polyglycolic acid sutures", Journal of Biomedical Materials Research, vol. 20, pp 613-632 (1986), the effect of annealing treatments on the tensile properties and hydrolytic degradative properties of polyglycolic acid sutures is tested by immersing the samples in a phosphate buffer solution (pH = 7.4) at 37°C for 7, 14, 21, and 28 days and by measuring the tensile properties after removal from the buffer solution.

The article of K.A. Athanasiou et al.: "Sterilization, toxicity, biocompatibility and clinical applications of polylactic acid/polyglycolic acid copolymers", Biomaterials, vol. 17, pp 93-102 (1996) relates to the sterilization, toxicity, biocompatibility and clinical applications of polylactic acid/polyglycolic acid copolymers. For test purposes, samples are incubated at 37°C in a buffer; the incubation period is not disclosed, however.

C.C. Chu and D.F. Williams: "The effect of gamma irradiation on the enzymatic degradation of polyglycolic acid absorbable sutures", Journal of Biomedical Materials Research, vol. 17, pp 1029-1040 (1983) study the effect of gamma irradiation on the enzymatic degradation of polyglycolic acid absorbable sutures. For evaluation purposes, the samples are incubated at 37°C for 3, 7, and 14 days.

It is the problem of the invention to provide a process for reducing the resorption period of hydrolyzable resorbable surgical suture material, whereby the decrease of tensile strength can be accelerated and the resorption period can be shortened, which allows exact adjustment of the desired resorption properties and which can be introduced into the manufacturing process without problems and at low cost.

This problem is solved by a process for reducing the resorption period of hydrolyzable resorbable surgical suture material having the features of Claim 1. Advantageous versions are given in the dependent claims.

The process according to the invention is suitable for treating hydrolyzable resorbable surgical suture material. The surgical suture material is incubated in a hydrolysis buffer having an index of pH in the range from 4 to 10, for a period in the range from 10 hours to 100 hours. The temperature is in the range from 30°C to 65°C.

The process according to the invention can be well integrated in the manufacturing process of surgical suture material. It is assumed that it works by attacking the molecular structure by means of a hydrolysis process (hydrolytic breakdown), which results in a faster degradation of the suture material, in vitro and in vivo. The resorption and the decrease of tensile strength in vitro and in vivo can be determined by selection of the process parameters. There is the tendency that the resorption period is shorter and the tensile strength decreases faster when the incubation step in the hydrolysis buffer is performed for a longer period of time or at a higher temperature. The same holds when the index of pH deviates more significantly from pH = 7, upwards or downwards. Suture material of polyglactin can be produced by means of the process according to the invention, its tensile strength in vivo five days after implantation being in the range from 10% to 90% of the initial tensile strength, and its tensile strength in vivo fourteen days after implantation amounting to 0% to 30% of the initial tensile strength. A typical value for the resorption period (i.e. the time period after which substance of the suture material cannot be detected in vivo any more) of suture material pre-treated according to the invention is, e.g., 35 days, whereas the resorption period of conventional "Vicryl" is about 70 days.

The process according to the invention can be applied to monofilament and to multifilament (i.e. braided) surgical suture material. In the latter case the incubating step preferably is performed after the yarn used for the surgical suture material has been braided to a thread and the thread has been stretched and annealed (i.e. at a temperature from, e.g., 80°C to 115°C treated by heat) and preferably has been submitted to a cleaning process in an organic solvent (scouring). The conditions for the annealing depend on the thickness of the thread. It is the object to obtain, after annealing and the consecutive process steps (including the incubating step in the hydrolysis buffer), finished suture material which has as high a knot ultimate strength (see below) as possible and the tensile strength of which decreases in a well-defined manner in vivo, i.e. after implantation.

After the incubating step the surgical suture material is preferably rinsed by means of water, which can be supported by the application of ultrasound, and dried thereafter. The drying step can be performed initially under vacuum conditions at room temperature and thereafter under vacuum conditions at increased temperature.

Thereafter the surgical suture material can be processed in the usual manner. In this way, it can be coated and subsequently pliabilized, whereupon it is preliminary stocked in cans. Later the final processing can be performed, wherein, e.g., a thread of surgical suture material is provided with a surgical needle and is wound. After packaging in a primary package, a sterilization is performed, e.g. by treatment with ethylene oxide gas. The primary package can be overwrapped with a secondary package, whereupon in case of gas sterilization a second sterilization is carried out. It is also possible to get along with a single sterilization, e.g., by means of irradiation (e.g., by a treatment using gamma rays, e.g. applying 25 kGy, or, e.g., by irradiation using electrons). A decrease of the tensile strength of the suture material due to the irradiation has to be taken into account when the conditions of incubation in the hydrolysis buffer are determined.

In general, suitable synthetic absorbable (resorbable) polymers that may be used for surgical suture material with the present invention include polymers selected from the group consisting of aliphatic polyanhydrides (described in U.S. Patent 4,757,128), aromatic polyanhydrides (described in U.S. Patent 5,264,540), radiation-stable polylactones (described in U.S. Patents 4,435,590, 4,510,295, 4,532,928 and 4,689,424), poly(esteranhydrides) (as described in U.S. patent application Serial No. 03/062,865 filed May 14, 1993 and assigned to Ethicon, Inc.), polyiminocarbonates, polyesters made by step growth polymerization, especially polyesters that are absorbable like those made from oxalic (described in U.S. Patent 4,141,087), malic, or tartaric acids, polyamides made by step growth or ring opening polymerization, nontoxic structural poly(aminoacids) or polypeptides made by the ring opening polymerization of N-carboxyanhydrides or by genetic engineering, poly(hydroxybutyrate), poly(hydroxy-butyrate-co-hydroxyvalerate), other bacterially derived polyesters (described in Lenz et al., Macromolecules 22, 1106 (1989); 23, 5059 (1990); 24, 5256 (1991); 25, 1852 (1992)), polyphosphazenes, polyesteramides like polymorpholinediones (described in U.S. Patents 4,441,496 and 4,916,209), and block copolymers of polyethylene glycol and polylactones (described in U.S. Patent 4,452,973). Preferably, the absorbable polymer is a synthetic polymer. The preferred synthetic absorbable polymers are derived from the class of monomers generally referred to in the art as lactone monomers (including acid equivalents of these monomers that may be used to form absorbable polymers). Examples of lactone monomers include glycolide, lactide, 1,4-dioxanone, trimethylene carbonate, δ-valerolactone, ε-caprolactone, 1,4-dioxepan-2-one, 1,5-dioxepan-2-one, and substituted equivalents of these compounds as well as the cyclic dimers of these compounds. Preferred are polymers containing two or more of these monomers including copolymers of glycolide and lactide; copolymers of caprolactone and glycolide; copolymers of glycolide and trimethylene carbonate; and terpolymers of glycolide, trimethylene and p-dioxanone. Also envisioned as suitable are random, block or graft copolymers of any of these lactone monomers and polymeric blends thereof.

In particular, appropriate surgical suture materials which can be submitted to the process according to the invention are, for example, copolymers of glycolide and lactide, in particular polyglactin 910, polyglecaprone, poly-p-dioxanone, mixtures of these substances, but also other hydrolyzable materials. Caprolactone/glycolide copolymers, polyglycolides, and glycolide/trimethylen carbonate/p-dioxanone copolymers are listed as further examples.

In the following the invention is explained in more detail by means of examples.

The resorbable surgical suture material according to the first examples consists of multifilament polyglactin 910. The process for treatment according to the invention, however, can also be carried out using monofilament material. Additional materials are described in Example 5.

For pre-manufacturing, yarn made of polyglactin 910 is supplied in cans. Yarn made of polyglactin should be stored under vacuum or in an inert gas atmosphere. After opening the cans, the yarn is uncoiled and, following a pre-determined braiding pattern, braided to a thread having a desired thickness. Thereafter, the thread is stretched and annealed. The annealing serves for increasing the crystallinity of the polymer and for preventing the suture material from shrinking during later manufacturing steps.

Table 1 shows the influence of the temperature applied during annealing onto several ultimate strengths, measured for a yarn having a nominal diameter of 0.1 mm after an annealing period of two hours. Here and in the following, straight ultimate strength is understood as the force when the test thread breaks in the stretched state. In order to determine the knot ultimate strength, the test thread is provided with a knot prior to the breaking test. In this case, the thread breaks at the knot. In absolute terms, the knot ultimate strength is smaller than the straight ultimate strength because the suture material is damaged by making the knot. The knot ultimate strength is important in practice because surgical suture material is generally knotted in a surgical operation. The 96h-in vitro ultimate strength is the straight ultimate strength which is measured after putting the test thread into a hydrolysis buffer having the index of pH 7.26 at a temperature of 50.5°C for a period of 96 hours. It gives an indication for the decrease of the tensile strength in vivo (i.e. after implantation of the suture material into the body) after 21 days.

The ultimate strengths in Table 1 are normalized to the values at a temperature of 113°C and were obtained as mean values of several single measurements. The ultimate strengths increase with increasing annealing temperature.

**Table 1**

| Influence of the temperature during annealing on several ultimate strengths | | | |
|---|---|---|---|
| Temperature during annealing [°C] | Straight ultimate strength [%] | Knot ultimate strength [%] | 96h-in vitro ultimate strength [%] |
| 113 | 100 | 100 | 100 |
| 100 | | 98,2 | 70,4 |
| 80 | 93,9 | 96,6 | 49,0 |

Basically, the conditions for annealing depend on the thickness of the thread. It is the object to obtain a finished suture material after annealing and the consecutive process steps (including incubation in the hydrolysis buffer) which has as high a knot ultimate strength as possible and the tensile strength of which in vivo, i.e. after implantation, decreases in a well-defined manner.

After annealing, a surface treatment by scouring follows. The scouring step can also be carried out prior to annealing. Up to here, the individual steps are familiar to the skilled person.

As the next step, the process according to the invention is carried out. Some examples serve for explanation purposes.

### Example 1

Braided threads having a nominal diameter of 0.05 mm, 0.07 mm, 0.10 mm, 0.15 mm, 0.20 mm, 0.30 mm, 0.35 mm or 0.40 mm (which, as described, had been annealed at an optimum temperature, e.g. 113°C, and had not been pre-irradiated) wound onto coils were put into an incubation bath, the lid of which was immediately closed. The incubation bath was filled with a hydrolysis buffer which already had its operating temperature when the coils were inserted.

The hydrolysis buffer included a buffer system of Na₂HPO₄ (50 mMol/l) and KH₂PO₄ (17 mMol/l) in aqueous solution. The pH index was 7.26.

The operating temperature was 50.5°C and was maintained essentially constant (about to ± 0,1 K) by means of a control circuit.

The threads can remain in the incubation bath for different periods. The resorption period and the decrease of tensile strength are influenced by this. Table 2 (see below) gives values for several ultimate strengths at incubation periods from 50 to 80 hours, compared to ultimate strenghts of threads which have not been treated in a hydrolysis buffer.

In a preferred embodiment, the threads remain for a period of about 41 hours to 48 hours in the incubation bath, wherein the exact period can be determined in the same way as described below in Example 2.

Thereafter, in the example, the coils with the threads were taken out of the incubation bath and were rinsed using running demineralized or distilled water. Then they were inserted in an ultrasound bath filled with demineralized or distilled water and were left there for about 30 minutes. For finishing the cleaning procedure, the coils were rinsed again under running demineralized or distilled water. The adhering water was shaken away.

Afterwards, the threads were dried on the coils. Initially they were pre-dried in a vacuum cabinet at room temperature for at least four hours. Then the coils with the threads were finally dried under vacuum conditions for at least 16 hours at about 50°C.

Table 2 presents ultimate strengths after different incubation periods, normalized to threads which had been incubated for 0 hours, i.e. which had not been treated in the hydrolysis buffer.

The straight ultimate strength and the knot ultimate strength are defined as explained for Table 1. The 24h-in vitro ultimate strength is the straight ultimate strength measured after incubating the test thread in a hydrolysis buffer having the index of pH 7.26 at a temperature of 50.5°C (i.e. the hydrolysis buffer used for incubating) for a period of 24 hours. It gives an indication for the decrease of the tensile strength in vivo (i.e. after implantation of the suture material in the body). Because of the normalization to 100%, the given values for the ultimate strengths are independent from the diameter of the respective test thread, and so the measured individual values for different thread diameters (after normalization to threads having the respective diameter incubated for 0 hours) can be averaged. Table 2 shows respective mean values of several individual measurements of threads having different diameters. The position where the respective measured test thread breaks is not significantly influenced by the fact that the threads were in the incubation bath when wound on coils, because the hydrolysis buffer is able to act onto the threads at virtually every surface position.

**Table 2**

| Influence of the incubation period on several ultimate strengths | | | |
|---|---|---|---|
| Incubation period [h] | Straight ultimate strength [%] | Knot ultimate strength [%] | 24h-in vitro ultimate strength [%] |
| 0 | 100 | 100 | 100 |
| 50 | 81,1 | 80,1 | 69,4 |
| 60 | 73,9 | 73,1 | 60,6 |
| 70 | 65,5 | 64,9 | 55,2 |
| 80 | 59,9 | 57,0 | 45,2 |

Table 2 indicates that the ultimate strengths decrease when the incubation period increases.

### Example 2

For braided threads having a nominal diameter of 0.04 mm, the period the threads, wound on coils, had to spend in an incubation bath with a hydrolysis buffer as in Example 1 at an operating temperature of 50.5°C was experimentally determined.

For this purpose, firstly the sample of the lot to be treated was incubated for 40 hours. After drying (and coating, see below), the ultimate strength of threads of the sample was measured, i.e. in two different ways. In one alternative, threads were used which had been provided with a knot but which had not been post-treated. In the test, such a thread breaks at the position of the knot (knot ultimate strength). In the other alternative, the ultimate strength was measured under linear tension conditions for threads which had been put for 24 hours at 50.5°C in an in vitro solution according to Example 1 (24h-in vitro ultimate strength). Depending on the values measured for the ultimate strength, the incubation period for the rest of the lot was determined as indicated in Table 3. The higher the ultimate strength of the sample, the higher the incubation period for the rest of the lot.

**Table 3**

| Determination of the incubation period for braided suture material of polyglactin 910 having a nominal diameter of 0.04 mm by means of ultimate strength measurements (mean values) of samples incubated for 40 hours | | |
|---|---|---|
| Knot ultimate strength [N] | 24h-in vitro ultimate strength [N] | Incubation period [h] |
| <0,539 | 0,55-1,05 | 32 |
| 0,54-0,59 | 0,55-1,05 | 36 |
| >0,60 | 0,55-1,05 | 40 |
| >0,60 | 1,05-1,13 | 44 |
| >0,60 | ≥1,14 | 48 |

After the incubating step, the threads wound on the coils were cleaned and dried, as indicated in Example 1.

### Example 3

The influence of the incubation temperature on the ultimate strength has been investigated for braided threads having a nominal diameter of 0.15 mm, 0.30 mm, 0.35 mm and 0.40 mm. Threads, wound on coils, were treated in the hydrolysis buffer according to Example 1 (index of pH 7.26) for 50 hours at a constant temperature.

Table 4 shows the ultimate strengths after treatment at different incubation temperatures, normalized to threads which were incubated at 50.5°C. The straight ultimate strength, the knot ultimate strength and the 24h-in vitro ultimate strength are defined as explained for Table 2. Because of the normalization to 100%, the given ultimate strength values are independent from the diameter of the respective test thread, so the measured individual values for different thread diameters (after normalization to threads having the respective diameter incubated at 50.5°C) can be averaged. Table 4 shows respective mean values for several individual measurements of threads having different diameters.

The ultimate strengths decrease when the incubation temperature increases.

**Table 4**

| Influence of the incubation temperature on several ultimate strengths | | | |
|---|---|---|---|
| Incubation temperature [°C] | Straight ultimate strength [%] | Knot ultimate strength [%] | 24h-in vitro ultimate strength [%] |
| 45,0 | 113,1 | 117,9 | 124,5 |
| 50,5 | 100 | 100 | 100 |
| 55,0 | 63,9 | 69,7 | 53,7 |

### Example 4

The influence of the index of pH of the hydrolysis buffer on the ultimate strength has been investigated for braided threads having a nominal diameter of 0.40 mm. Threads, wound on coils, were treated in a hydrolysis buffer for 50 hours at an incubation temperature of 50.5°C. A phosphate buffer system having a total concentration of the anions of 67 mMol/l and an index of pH adjusted to the desired value was used as hydrolysis buffer.

Table 5 gives the ultimate strengths after treatment at different indices of pH, normalized to threads which were incubated at an index of pH of 7.26. The straight ultimate strength, the knot ultimate strength and the 24h-in vitro ultimate strength are defined as explained for Table 2. Table 5 shows the respective mean values of several individual measurements for threads of the nominal diameter 0.40 mm.

**Table 5**

| Influence of the index of pH of the hydrolysis buffer on several ultimate strengths | | | |
|---|---|---|---|
| Index of pH | Straight ultimate strength [%] | Knot ultimate strength [%] | 24h-in vitro ultimate strength [%] |
| 7,26 | 100 | 100 | 100 |
| 8,27 | 95,7 | 89,4 | 98,3 |

For incubation in the more alkaline hydrolysis buffer, the ultimate strengths are smaller.

After incubating, cleaning and drying, the further processing of the surgical suture material can be carried out in the conventional manner. Usually, a coating is applied to braided threads. Thereafter the threads are pliabilized. Then the threads, packaged in cans, can be preliminarily stocked.

The final processing of the surgical suture material is performed in known manner as well. The threads can be provided with needles and can be wound. After packaging, e.g. in a foil serving as primary package, a sterilization can be carried out, e.g. by irradiation or by using ethylene oxide. A sterilization, e.g. by irradiation, can also be performed after overwrapping with a secondary package.

As a special feature it should be emphasized that the dryness of the packaging material of the primary package should be as great as possible. In order to achieve that, the wound threads, which have been provided with a needle, if applicable, are put into, e.g., a foil envelope which is not yet sealed. In this state a drying process under vacuum conditions at a temperature of, e.g., 45°C to 65°C for a period of, e.g., 20 to 80 hours is carried out. Thereafter, the packages are sealed so that humid air cannot enter any more. A further wrapping serves as secondary package. If sterilization by irradiation is applied, a single sterilization step is sufficient which is to be performed after attaching the secondary package.

If irradiation is used for sterilization, e.g. by a radiation dose of 25 kGy, it has to be noted that the tensile strength, in particular the 24h-in vitro ultimate strength, of irradiated suture material is smaller than that of suture material which has not been irradiated. The incubation conditions in the hydrolysis buffer have to be adjusted to the subsequent sterilizing irradiation in order that the 24h-in vitro ultimate strength of the finished, sterilized suture material has the desired values. I.e., in such case the decrease of tensile strength results in part from the incubation in the hydrolysis buffer and in part from the irradiation used for sterilization.

Table 6 gives indications for the effect due to the irradiation. It shows the straight ultimate strength, the knot ultimate strength and the 24h-in vitro ultimate strength (defined as explained in Example 1) for threads which were submitted to several radiation doses (⁶⁰Co gamma radiation), normalized to non-irradiated threads. Prior to irradiation, the threads investigated were not incubated in a hydrolysis buffer. The given values are respective mean values for several individual measurements of braided threads made of polyglactin 910 having a nominal diameter of 0.30 mm.

**Table 6**

| Influence of irradiation on several ultimate strengths of suture material which was not incubated in a hydrolysis buffer | | | |
|---|---|---|---|
| Radiation dose [kGy] | Straight ultimate strength [%] | Knot ultimate strength [%] | 24h-in vitro ultimate strength [%] |
| 0 | 100 | 100 | 100 |
| 25 | 80,2 | 84,0 | 71,7 |
| 50 | 68,9 | 70,5 | 48,6 |

The following Example 5 demonstrates that the incubation according to the invention in a hydrolysis buffer can be carried out with hydrolyzable surgical suture materials different from polyglactin 910 as well.

### Example 5

It was the object to determine the straight ultimate strength (as defined in Example 1 and in connection with Table 1) of different commercially available suture materials, all of them having a nominal diameter of 0.4 mm, after they had been subjected to a hydrolysis buffer for different incubation periods (50 h, 60 h, 70 h, and 80 h). The corresponding untreated samples (i.e. incubation period 0 h) serve for comparison purposes. The phosphate buffer system of Example 1 (index of pH 7.27) was used as hydrolysis buffer, at a temperature of 50.5°C.

The following suture materials were examined:
(a) BIOSYN (a p-dioxanon/trimethylene carbonate/glycolide copolymer
(b) MONOCRYL (a caprolactone/glycolide copolymer)
(c) POLYSORB (a glycolide/lactide copolymer in the ratio 90:10)
(d) DEXON II (a glycolide homopolymer or polyglycolide)
The given designations are trademarks.

40 pieces of 25 cm length each were used for each of the suture materials (a) to (d). In each case, the 40 pieces were subdivided into four groups of 10 pieces each. Each group was put to a glass vessel containing 175 ml of hydrolysis buffer. The glass vessels were tightly closed and were placed into a water bath of 50.5°C for several preselected incubation periods (i.e. 50 h, 60 h, 70 h, and 80 h). After termination of a preselected incubation period, for each of the suture materials (a) to (d) a glass vessel was taken out.

After removal of the samples from the water bath, the hydrolysis buffer was drained from the respective glass vessel, and the suture material was rinsed in demineralized water and placed into an ultrasonic bath for 15 minutes. Then the suture material was rinsed again in demineralized water, placed on clean absorbent paper to remove excess water, and vacuum dried at room temperature for at least four hours. The suture material was then finally dried for 12 hours at 50°C under vacuum.

Afterwards the straight ultimate strength was measured for each of the suture materials (a) to (d) and each of the preselected incubation periods, using each of the 10 pieces, so that a mean value could be calculated from the 10 individual values in each case. For comparison purposes, the straight ultimate strength of untreated samples, which were not exposed to the hydrolysis buffer (incubation period 0 h), was determined for each of the suture materials (a) to (d).

Table 7 shows the results for the straight ultimate strength, the respective mean values for incubated suture material being related to the corresponding values for untreated suture material (incubation period 0 h, corresponding to straight ultimate strength of 100%).

**Table 7**

| Influence of incubation period on the straight ultimate strength of several suture materials | | | | | |
|---|---|---|---|---|---|
| Suture material | Straight ultimate strength [%] for incubation period of | | | | |
| | 0 h | 50 h | 60 h | 70 h | 80 h |
| (a) | 100 | 82.2 | 76.9 | 67.8 | 65.3 |
| (b) | 100 | 52.2 | 44.4 | 33.2 | 30.1 |
| (c) | 100 | 76.8 | 70.6 | 58.5 | 54.2 |
| (d) | 100 | 81.7 | 71.1 | 56.6 | 52.7 |

## Claims

1. Process for reducing the resorption period of hydrolyzable resorbable surgical suture material, wherein the surgical suture material is incubated in a hydrolysis buffer, having an index of pH in the range from 4 to 10, for a period in the range from 10 hours to 100 hours at a temperature in the range from 30°C to 65°C.

2. Process according to Claim 1, **characterized in that** the incubation is performed after the yarn used for the surgical suture material has been braided to form a thread, the thread has been stretched and annealed, preferably at a temperature in the range from 70°C to 120°C, and preferably has been submitted to a cleaning process in an organic solvent.

3. Process according to Claim 1 or 2, **characterized in that** after the incubating step the surgical suture material is cleaned by means of water and is subsequently dried.

4. Process according to Claim 3, **characterized in that** the drying step is performed initially at room temperature, using vacuum, and subsequently at higher temperature, using vacuum.

5. Process according to one of Claims 1 to 4, **characterized in that** the index of pH of the hydrolysis buffer is in the range from 5 to 9.

6. Process according to one of Claims 1 to 4, **characterized in that** the index of pH of the hydrolysis buffer is in the range from 6 to 8.

7. Process according to one of Claims 1 to 4, **characterized in that** the index of pH of the hydrolysis buffer is in the range from 7.0 to 7.5.

8. Process according to Claim 7, **characterized in that** the hydrolysis buffer comprises a phosphate buffer system having a concentration in the range from 50 mMol/l to 100 mMol/l.

9. Process according to one of Claims 1 to 8, **characterized in that** during the incubating step the temperature is in the range from 40°C to 60°C.

10. Process according to one of Claims 1 to 8, **characterized in that** during the incubating step the temperature is in the range from 47°C to 53°C.

11. Process according to one of Claims 1 to 8, **characterized in that** during the incubating step the temperature is in the range from 50°C to 60°C.

12. Process according to one of Claims 1 to 11, **characterized in that** the period for the incubating step is in the range from 30 hours to 70 hours.

13. Process according to one of Claims 1 to 11, **characterized in that** the period for the incubating step is in the range from 40 hours to 55 hours.

14. Process according to one of Claims 1 to 11, **characterized in that** the period for the incubating step is in the range from 10 hours to 50 hours.

15. Process according to one of Claims 1 to 14, **characterized in that** the period for the incubating step is determined by firstly incubating a sample of the lot of surgical suture material to be incubated at the temperature to be used for a pre-selected period and by measuring thereafter the tensile strength thereof, which yields the period the rest of the lot of the surgical suture material is to be incubated by means of an experimentally determined, relation.

16. Process according to one of Claims 1 to 15, **characterized in that** the surgical suture material is irradiated.

17. Process according to one of Claims 1 to 16, **characterized in that** the surgical suture material includes a glycolide/lactide copolymer, in particular polyglactin 910.

18. Process according to one of Claims 1 to 17, **characterized in that** the surgical suture material includes poly-p-dioxanone.

19. Process according to one of Claims 1 to 18, **characterized in that** the surgical suture material includes a caprolactone/glycolide copolymer.

20. Process according to one of Claims 1 to 19, **characterized in that** the surgical suture material includes a polyglycolide.

21. Process according to one of Claims 1 to 20, **characterized in that** the surgical suture material includes a glycolide/trimethylene carbonate/p-dioxanone copolymer.

22. Process according to one of Claims 1 to 21, **characterized in that** the surgical suture material includes polyglecaprone.

## Patentansprüche

1. Verfahren zum Reduzieren der Resorptionsdauer von hydrolysierbarem resorbierbarem chirurgischem Nahtmaterial, wobei das chirurgische Nahtmaterial in einem Hydrolysepuffer mit einem pH-Wert im Bereich von 4 bis 10 für eine Zeitdauer im Bereich von 10 Stunden bis 100 Stunden bei einer Temperatur im Bereich von 30 °C bis 65 °C inkubiert wird.

2. Verfahren nach Anspruch 1, **dadurch gekennzeichnet, daß** das Inkubieren durchgeführt wird, nachdem das für das chirurgische Nahtmaterial verwendete Garn zu einem Faden geflochten und der Faden verstreckt und getempert, vorzugsweise bei einer Temperatur im Bereich von 70 °C bis 120 °C, und vorzugsweise einem Waschprozeß in einem organischen Lösungsmittel unterzogen worden ist.

3. Verfahren nach Anspruch 1 oder 2, **dadurch gekennzeichnet, daß** das chirurgische Nahtmaterial nach dem Inkubieren mit Wasser gereinigt und anschließend getrocknet wird.

4. Verfahren nach Anspruch 3, **dadurch gekennzeichnet, daß** das Trocknen zunächst im Vakuum bei Raumtemperatur und anschließend im Vakuum bei erhöhter Temperatur erfolgt.

5. Verfahren nach einem der Ansprüche 1 bis 4, **dadurch gekennzeichnet, daß** der pH-Wert des Hydrolysepuffers im Bereich von 5 bis 9 liegt.

6. Verfahren nach einem der Ansprüche 1 bis 4, **dadurch gekennzeichnet, daß** der pH-Wert des Hydrolysepuffers im Bereich von 6 bis 8 liegt.

7. Verfahren nach einem der Ansprüche 1 bis 4, **dadurch gekennzeichnet, daß** der pH-Wert des Hydrolysepuffers im Bereich von 7,0 bis 7,5 liegt.

8. Verfahren nach Anspruch 7, **dadurch gekennzeichnet, daß** der Hydrolysepuffer ein Phosphat-Puffersystem mit einer Konzentration im Bereich von 50 mMol/l bis 100 mMol/l aufweist.

9. Verfahren nach einem der Ansprüche 1 bis 8, **dadurch gekennzeichnet, daß** beim Inkubieren die Temperatur im Bereich von 40 °C bis 60 °C liegt.

10. Verfahren nach einem der Ansprüche 1 bis 8, **dadurch gekennzeichnet, daß** beim Inkubieren die Temperatur im Bereich von 47 °C bis 53 °C liegt.

11. Verfahren nach einem der Ansprüche 1 bis 8, **dadurch gekennzeichnet, daß** beim Inkubieren die Temperatur im Bereich von 50 °C bis 60 °C liegt.

12. Verfahren nach einem der Ansprüche 1 bis 11, **dadurch gekennzeichnet, daß** die Zeitdauer für das Inkubieren im Bereich von 30 Stunden bis 70 Stunden liegt.

13. Verfahren nach einem der Ansprüche 1 bis 11, **dadurch gekennzeichnet, daß** die Zeitdauer für das Inkubieren im Bereich von 40 Stunden bis 55 Stunden liegt.

14. Verfahren nach einem der Ansprüche 1 bis 11, **dadurch gekennzeichnet, daß** die Zeitdauer für das Inkubieren im Bereich von 10 Stunden bis 50 Stunden liegt.

15. Verfahren nach einem der Ansprüche 1 bis 14, **dadurch gekennzeichnet, daß** die Zeitdauer für das Inkubieren bestimmt wird, indem zunächst eine Probe des zu inkubierenden Loses an chirurgischem Nahtmaterial bei der zu verwendenden Temperatur für eine vorgewählte Zeitdauer inkubiert wird und danach deren Reißkraft gemessen wird, woraus sich mittels einer experimentell ermittelten Beziehung die Zeitdauer ergibt, für die der Rest des Loses an chirurgischem Nahtmaterial zu inkubieren ist.

16. Verfahren nach einem der Ansprüche 1 bis 15, **dadurch gekennzeichnet, daß** das chirurgische Nahtmaterial bestrahlt wird.

17. Verfahren nach einem der Ansprüche 1 bis 16, **dadurch gekennzeichnet, daß** das chirurgische Nahtmaterial ein Glykolid/-Lactid-Copolymer aufweist, insbesondere Polyglactin 910.

18. Verfahren nach einem der Ansprüche 1 bis 17, **dadurch gekennzeichnet, daß** das chirurgische Nahtmaterial Poly-p-dioxanon aufweist.

19. Verfahren nach einem der Ansprüche 1 bis 18, **dadurch gekennzeichnet, daß** das chirurgische Nahtmaterial ein Caprolacton/Glykolid-Copolymer aufweist.

20. Verfahren nach einem der Ansprüche 1 bis 19, **dadurch gekennzeichnet, daß** das chirurgische Nahtmaterial ein Polyglykolid aufweist.

21. Verfahren nach einem der Ansprüche 1 bis 20, **dadurch gekennzeichnet, daß** das chirurgische Nahtmaterial ein Glykolid/-Trimethylencarbonat/p-dioxanon-Copolymer aufweist.

22. Verfahren nach einem der Ansprüche 1 bis 21, **dadurch gekennzeichnet, daß** das chirurgische Nahtmaterial Polyglecaprone aufweist.

## Revendications

1. Procédé de réduction du temps de résorption d'un matériau de suture chirurgicale résorbable par hydrolyse, dans lequel le matériau de suture chirurgicale est incubé dans un tampon d'hydrolyse, ayant un indice de pH dans la plage de 4 à 10, pendant une durée dans la plage de 10 à 100 heures à une température dans la plage de 30 à 65°C.

2. Procédé selon la revendication 1, **caractérisé en ce que** l'incubation est réalisée après que le fil utilisé à titre de matériau de suture chirurgicale a été tressé pour former un fil tressé, le fil tressé a été étiré et recuit, de préférence, à une température dans la plage de 70 à 120°C, et a, de préférence, été soumis à un procédé de nettoyage dans un solvant organique.

3. Procédé selon la revendication 1 ou 2, **caractérisé en ce que**, après l'étape d'incubation, le matériau de suture chirurgicale est nettoyé à l'eau et est ensuite séché.

4. Procédé selon la revendication 3, **caractérisé en ce que** l'étape de séchage est d'abord mise en oeuvre à la température ambiante, en utilisant un vide, et par la suite à une température plus élevée, en utilisant un vide.

5. Procédé selon l'une des revendications 1 à 4, **caractérisé en ce que** l'indice de pH du tampon d'hydrolyse est dans la plage de 5 à 9.

6. Procédé selon l'une des revendications 1 à 4, **caractérisé en ce que** l'indice de pH du tampon d'hydrolyse est dans la plage de 6 à 8.

7. Procédé selon l'une des revendications 1 à 4, **caractérisé en ce que** l'indice de pH du tampon d'hydrolyse est dans la plage de 7,0 à 7,5.

8. Procédé selon la revendication 7, **caractérisé en ce que** le tampon d'hydrolyse comprend un système de tampon phosphate à une concentration dans la plage de 50 à 100 mMoles/l.

9. Procédé selon l'une des revendications 1 à 8, **caractérisé en ce que**, pendant l'étape d'incubation, la température est dans la plage de 40 à 60°C.

10. Procédé selon l'une des revendications 1 à 8, **caractérisé en ce que**, pendant l'étape d'incubation, la température est dans la plage de 47 à 53°C.

11. Procédé selon l'une des revendications 1 à 8, **caractérisé en ce que**, pendant l'étape d'incubation, la température est dans la plage de 50 à 60°C.

12. Procédé selon l'une des revendications 1 à 11, **caractérisé en ce que** la durée de l'étape d'incubation est dans la plage de 30 à 70 heures.

13. Procédé selon l'une des revendications 1 à 11, **caractérisé en ce que** la durée de l'étape d'incubation est dans la plage de 40 à 55 heures.

14. Procédé selon l'une des revendications 1 à 11, **caractérisé en ce que** la durée de l'étape d'incubation est dans la plage de 10 à 50 heures.

15. Procédé selon l'une des revendications 1 à 14, **caractérisé en ce que** la durée de l'étape d'incubation est déterminée en incubant d'abord un échantillon du lot de matériau de suture chirurgicale qui doit être incubé à la température qui doit être utilisée pendant une durée présélectionnée et en mesurant ensuite sa tension de rupture, qui donne la durée pendant laquelle le reste du lot du matériau de suture chirurgicale doit être incubé par une relation déterminée par expérimentation.

16. Procédé selon l'une des revendications 1 à 15, **caractérisé en ce que** le matériau de suture chirurgicale est irradié.

17. Procédé selon l'une des revendications 1 à 16, **caractérisé en ce que** le matériau de suture chirurgicale comprend un copolymère de glycolide/lactide, en particulier, la Polyglactine 910.

18. Procédé selon l'une des revendications 1 à 17, **caractérisé en ce que** le matériau de suture chirurgicale comprend une poly-p-dioxanone.

19. Procédé selon l'une des revendications 1 à 18, **caractérisé en ce que** le matériau de suture chirurgicale comprend un copolymère de caprolactone/glycolide.

20. Procédé selon l'une des revendications 1 à 19, **caractérisé en ce que** le matériau de suture chirurgicale comprend un polyglycolide.

21. Procédé selon l'une des revendications 1 à 20, **caractérisé en ce que** le matériau de suture chirurgicale comprend un copolymère de glycolide/carbonate de triméthylène/p-dioxanone.

22. Procédé selon l'une des revendications 1 à 21, **caractérisé en ce que** le matériau de suture chirurgicale comprend une polyglecaprone.
